Europäisches Patentamt

(19) European Patent Office

Office européen des brevets

(11) Veröffentlichungsnummer : **0 033 000**
**B1**

(12) **EUROPÄISCHE PATENTSCHRIFT**

(45) Veröffentlichungstag der Patentschrift :
23.05.84

(21) Anmeldenummer : 80107882.5

(22) Anmeldetag : 13.12.80

(51) Int. Cl.³ : **C 07 G 7/00, A 61 K 37/02,**
**A 61 K 39/00, G 01 N 33/50,**
**G 01 N 33/68**

(54) **Neues Protein PP15, ein Verfahren zu seiner Herstellung sowie seine Verwendung.**

(30) Priorität : 31.12.79 DE 2952792

(43) Veröffentlichungstag der Anmeldung :
05.08.81 Patentblatt 81/31

(45) Bekanntmachung des Hinweises auf die Patenterteilung : 23.05.84 Patentblatt 84/21

(84) Benannte Vertragsstaaten :
**AT CH DE FR GB IT LI**

(56) Entgegenhaltungen :
**EP-A- 0 009 715**
**DE-A- 2 720 704**
**DE-A- 2 854 759**

(73) Patentinhaber : **BEHRINGWERKE AKTIENGESELL-SCHAFT**
**Postfach 1140**
**D-3550 Marburg/Lahn (DE)**

(72) Erfinder : **Bohn, Hans, Dr.**
**Oberer Eichweg 26**
**D-3550 Marburg 1 (DE)**

(74) Vertreter : **Meyer-Dulheuer, Karl-Hermann, Dr. et al**
**HOECHST Aktiengesellschaft Zentrale Patentabteilung Postfach 80 03 20**
**D-6230 Frankfurt/Main 80 (DE)**

# 0 033 000

**Beschreibung**

Die Erfindung betrifft ein neues immunsuppressiv wirksames Protein ($PP_{15}$) und ein Verfahren zu dessen Herstellung aus menschlichen Plazenten.

Aus DE-OS 27 20 704 ist ein aus Plazenta erhältliches Glykoprotein bekannt, das sich jedoch in den kennzeichnenden Parametern und seinen proteolytischen Eigenschaften von $PP_{15}$ unterscheidet.

Gegenstand der Erfindung ist das Protein $PP_{15}$, gekennzeichnet durch

a) einen Kohlenhydratanteil von $3,35 \pm 0,9\%$, bestehend aus $2,8 \pm 0,5\%$ Hexosen, $0,3 \pm 0,2\%$ Hexosaminen, $0,05 \pm 0,05\%$ Fucose und $0,20 \pm 0,15\%$ Neuraminsäure ;

b) einen Sedimentationskoeffizienten $S_{20,w}^0$ von $2,9 \pm 0,2$ S ;

c) ein in der Ultrazentrifuge bestimmtes Molekulargewicht von $30\,700 \pm 3\,200$ ;

d) einen Extinktionskoeffizienten $E_{1cm}^{1\%}$ (280 nm) von $14,2 \pm 1,0$ und

e) eine elektrophoretische Beweglichkeit im Bereich des Albumins sowie

f) einen isoelektischen Punkt von $4,4 \pm 0,1$.

Zur Erläuterung der kennzeichnenden Merkmale des Proteins sei folgendes ausgeführt :

Der Sedimentationskoeffizient wurde in einer analytischen Ultrazentrifuge der Firma Beckman (Spinco-Apparatur, Modell E) bei 60000 UpM in Doppelsektorzellen mit Hilfe der UV-Scanner Technik bei 280 nm bestimmt. Als Lösungsmittel diente ein 0,05 M Phosphatpuffer (pH 6,8), der 0,2 Mol/l NaCl enthielt. Die Proteinkonzentration wurde auf eine optische Dichte von etwa 3 eingestellt. Der Sedimentationskoeffizient wurde auf die Basis von Wasser bei 20 °C umgerechnet.

Zur Ermittlung des Molekulargewichts in der Ultrazentrifuge wurde die Sedimentationsgleichgewichtsmethode herangezogen. Die Konzentration des Proteins ist dabei auf eine optische Dichte von etwa 1,0 eingestellt worden. Die Bestimmung wurde bei 9000 UpM vorgenommen. Die Registrierung erfolgte mit UV-Optik bei 280 nm unter Einsatz des photoelektrischen Scanners.

Zur Bestimmung des Extinktionskoeffizienten wurde die Substanz 0,10 %ig in destilliertem Wasser gelöst.

Die elektrophoretische Beweglichkeit wurde in der Mikromodifikation mit dem Gerät Microzone R 200 von Beckman Instruments auf Zelluloseacetatfolien (Fa. Sartorius) unter Verwendung von Natriumdiäthylbarbiturat-Puffer, pH 8,6, bestimmt.

Der isoelektrische Punkt wurde mit einer Säule (440 ml) der Firma LKB, Stockholm, bestimmt. Das bei der Untersuchung von $PP_{15}$ verwendete Ampholin[R]-Gemisch hatte einen pH-Bereich von 3-5.

Die Bestimmung der Kohlenhydrate erfolgte nach der von H.E. Schultze, R. Schmidtberger, H. Haupt, Biochem. Z., 329, Seite 490 (1958), beschriebenen Methode.

Die Aminosäurenanalyse wurde nach S. Moore, D.H. Spackmann, H.W. Stein, Anal. Chem. [30], Seite 1185 (1958), unter Verwendung des Flüssigkeitschromatographen Multichrom B der Firma Beckman durchgeführt.

Cystin wurde nach Oxydation des Proteins mit Perameisensäure (S. Moore et al., Anal. Chem. [238], Seite 235 (1963) als Cysteinsäure bestimmt. Der Tryptophangehalt ist direkt photometrisch nach H. Edelhoch, Biochemistry 6, Seite 1948 (1967) ermittelt worden.

Tabelle I enthält das Ergebnis der Aminosäurenanalyse von $PP_{15}$.

Tabelle 1

Aminosäurenzusammensetzung von $PP_{15}$
(Reste pro 100 Reste in Mol.%)

|  |  | VK % |
| --- | --- | --- |
| Lysin | 4,74 | 3,30 |
| Histidin | 3,81 | 5,43 |
| Arginin | 1,62 | 3,43 |
| Asparaginsäure | 13,39 | 5,08 |
| Threonin | 3,85 | 5,35 |
| Serin | 6,38 | 2,81 |
| Glutaminsäure | 13,43 | 5,32 |
| Prolin | 4,35 | 14,25 |
| Glycin | 6,87 | 2,13 |
| Alanin | 6,51 | 8,26 |
| Cystin 1/2 | 2,48 | 4,55 |
| Valin | 2,29 | 15,67 |
| Methionin | 2,87 | 10,86 |
| Isoleucin | 8,39 | 8,18 |
| Leucin | 8,18 | 6,72 |
| Tyrosin | 2,09 | 8,49 |
| Phenylalanin | 6,27 | 2,27 |
| Tryptophan | 2,51 | 6,81 |

PP$_{15}$ hat folgende Eigenschaften, die sich zu seiner Isolierung verwenden lassen.

1) Mit Ammoniumsulfat wird es bei pH 7,0 und 30-60 % Sättigung aus wässrigen Lösungen gefällt.

2) Mit wasserlöslichen Acridinbasen, z. B. 2-Äthoxi-6,9-diaminoacridinlactat Rivanol[R] wird es bei pH-Werten zwischen 4-9 und einer Konzentration der Base von 0,2 bis 0,8 % w/v präzipitiert.

3) Unter den Bedingungen einer Euglobulinfällung, nämlich durch Einstellen eines pH-Wertes von 5-6, in einer verdünnten Pufferlösung wird es nicht präzipitiert.

4) In der präparativen Elektrophorese wandert es im Bereich des Albumins.

5) Bei der Gelfiltration mit Sephadex[R] verhält es sich wie Proteine mit Molekulargewichten von 10 000 bis 50 000.

6) Es läßt sich an schwach basische Ionenaustauscher wie beispielsweise DEAE-Cellulose oder DEAE-Sephadex bei einer Leitfähigkeit von 0-2 mS und einem pH-Wert von etwa pH 7 bis 9 binden.

7) An Hydroxylapatit wird es bei Verwendung einer etwa 0,01 M Phosphat-Pufferlösung im pH-Bereich von 6-8 nicht adsorbiert.

Gegenstand der Erfindung ist ferner ein Verfahren zur Gewinnung des PP$_{15}$, dadurch gekennzeichnet, daß eine Lösung, die dieses Protein enthält, unter Verwendung der obenstehenden Eigenschaften fraktioniert wird.

Durch eine zweckmäßige Kombination der genannten Maßnahmen, die eine Anreicherung des PP$_{15}$ oder eine Abtrennung dieses Proteins von anderen Proteinen bewirken, kann das PP$_{15}$ isoliert in praktisch reiner Form gewonnen werden.

Neben Ammoniumsulfat können selbstverständlich auch andere in der präparativen Biochemie üblicherweise eingesetzten Neutralsalze zur Ausfällung des PP$_{15}$ verwendet werden. Neben einer Acridinbase ist auch ein wasserlösliches Derivat einer Chinolinbase, wie sie für Proteinfraktionierungen bekannt sind, im Rahmen des erfindungsgemäßen Verfahrens einsetzbar. Entsprechend seinem elektrophoretischen Verhalten wie seinem Molekulargewicht sind zur Isolierung des Proteins auch andere Maßnahmen brauchbar, die geeignet sind, ein Globulin mit Albuminbeweglichkeit von anderen Proteinen abzutrennen. Hierzu können die verschiedenen Methoden der Gelfiltration, Gelchromatographie oder Ultrafiltration oder auch die Eigenschaft des PP$_{15}$ an schwach basische Ionenaustauscher gebunden und hiervon wieder eluiert werden zu können, verwendet werden.

Demzufolge ist der Gegenstand der vorliegenden Erfindung in den einzelnen Schritten zur Anreicherung des PP$_{15}$ und in dem durch Kombination der Maßnahmen zur Anreicherung sich ergebenden Verfahren zur Reinigung des PP$_{15}$ zu sehen.

Das Verfahren zur Anreicherung ist gekennzeichnet durch die Anwendung mindestens einer der Maßnahmen 1 bis 7 oder deren chemischen oder biochemischen präparativen Äquivalente.

PP$_{15}$ hat immunsuppressive Eigenschaften. Zur Messung der inhibitorischen Wirksamkeit auf die Lymphozytentransformation in vitro wurde PP$_{15}$ in den Mengen 150, 100, 50, 10,1 und 0,1 µg MLC-Kulturen zugesetzt. Nach sechs Tagen wurde die eingebaute Menge von C 14-Thymidin dieser Kulturen und Kontrollkulturen, denen kein PP$_{15}$ zugesetzt war, gemessen. Das Protein PP$_{15}$ zeigte in den Dosierungen 1-150 µg/Kultur eine signifikante inhibitorische Wirksamkeit.

Die Erfindung wird am nachstehenden Beispiel erläutert :

## Beispiel

### A) Extraktion der Plazenten und Fraktionierung des Extraktes mit Rivanol und Ammoniumsulfat

1000 kg tiefgefrorene menschliche Plazenten werden im Schneidmischer zerkleinert und mit 1000 l einer 0,4 %igen (w/w) Kochsalzlösung extrahiert. Der Extrakt wird, nach Abtrennung des Geweberückstandes durch Zentrifugation, mit 20 %iger (w/w) Essigsäure auf pH 6,0 eingestellt und unter Rühren mit 200 l einer 3 %igen (w : w) Lösung von 2-Äthoxy-6,9-Diaminoacridin-Lactat (Rivanol[R], Hoechst AG) versetzt. Den durch Zentrifugation abgetrennten Niederschlag versetzt man mit 500 l einer 2,5 %igen (w : w) NaCl-Lösung, rührt 4 Stunden und zentrifugiert vom abgeschiedenen Chlorid des 2-Äthoxy-6,9-Diaminoacridins ab. Die Lösung versetzt man unter Rühren langsam mit soviel festem Ammoniumsulfat, daß eine Endkonzentration von 30 % (w/v) erreicht wird, wodurch PP$_{15}$ zusammen mit anderen Proteinen ausfällt. Der Niederschlag wird abzentrifugiert. Man erhält etwa 4,5 kg einer feuchten Paste, die im Nachfolgenden als Fraktion A bezeichnet wird.

### B) Gelfiltration an Sephadex G-150

1500 g der Fraktion A werden in Wasser gelöst und gegen einen 0,01 M Tris-HCl-Puffer (pH 8,0), der 0,05 % NaN$_3$ enthält (Pufferlösung I) dialysiert. Die zurückbleibende Lösung wird auf eine mit Sephadex G-150 gefüllte Säule (60 × 56 cm) aufgetragen und mit Pufferlösung I eluiert. Die Eluate, die Proteine mit Molekulargewichten zwischen 10 000 bis 50 000 enthalten, werden gesammelt und als Fraktion B bezeichnet.

### C) Chromatographie an DEAE-Cellulose

Fraktion B wird an DEAE-Cellulose (Säule 10 × 28 cm) adsorbiert. Man spült die Säule mit Pufferlösung I und eluiert mit 0,85 %iger (w : v) Kochsalzlösung solange, bis im Durchlauf mit Trichloressigsäure keine Fällung mehr entsteht. Aus dem Eluat werden die Proteine durch Zusatz von soviel Ammoniumsulfat, daß die Konzentration 30 % (w/v) beträgt, ausgefällt. Der Niederschlag wird abzentrifugiert (Fraktion C).

### D) Euglobulinfällung

Fraktion C wird in Wasser gelöst und gegen Pufferlösung I dialysiert. Man stellt die Lösung durch Zugabe von 2 N Essigsäure unter Rühren auf pH 5,5 ein. Der Niederschlag, der im wesentlichen nur Begleitproteine enthält, wird abzentrifugiert. Der Überstand wird gegen einen 0,1 M Ammoniumbicarbonatpuffer dialysiert (Fraktion D).

### E) Präparative Zonenelektrophorese

Mit der präparativen Zonenelektrophorese wurde weitergereinigt. Als Puffer wird dabei eine 0,1 M Ammoniumbicarbonatlösung verwendet. Fraktion D wird hierzu in eine Apparatur zur präparativen Elektrophorese, wie sie beispielsweise von N. Heimburger und R. Schmidtberger in Behringwerke-Mitteilungen, Heft 43, Seite 83 ff., insbesondere auf Seite 119-120, beschrieben wird, eingetragen. Bei dem Gerät handelt es sich um die horizontale Anordnung einer Trägerelektrophorese in einem offenen Trog, in welchem das Trägermaterial zur Abführung der bei der Elektrophorese auftretenden Joule'schen Wärme auf unter 10 °C gekühlt wird. Als Trägermaterial dienen gegenüber Proteinen indifferente Substanzen, vorteilhaft Polyvinylchlorid, oder dessen Copolymerisate in Form eines feinen Granulats. Es ist empfehlenswert, die Elektrophorese bei einer Feldstärke von 4-6 Volt/cm vorzunehmen. Das Protein $PP_{15}$ wandert im elektrischen Feldt schneller als die $\alpha_1$-Globuline. Die das neue Protein enthaltende Zone wird nach der Auftrennung herausgeschnitten und mit Wasser eluiert. Die Eluate werden anschließend lyophilisiert oder auf einem Ultrafilter eingeengt (Fraktion E).

### F) Chromatographie an Hydroxylapatit

Zur Weiterreinigung wurde an Hydroxylapatit (Säule 4 × 20 cm) unter Verwendung eines 0,01 M Phosphatpuffers, pH 6,8, chromatographiert. Das Protein $PP_{15}$ erscheint dabei im Durchlauf, während die noch vorhandenen Begleitproteine an Hydroxylapatit adsorbiert werden. Der Durchlauf, der das Protein $PP_{15}$ in reiner Form enthält, wird auf einem Ultrafilter eingeengt, gegen Wasser dialysiert und lyophilisiert.

**Ansprüche** (für die Vertragsstaaten : CH, DE, FR, GB, IT, LI)

1. Protein $PP_{15}$ gekennzeichnet durch
   a) einen Kohlenhydratanteil von 3,35 ± 0,9 %, bestehend aus 2,8 ± 0,5 % Hexosen, 0,3 ± 0,2 % Hexosaminen, 0,05 ± 0,05 % Fucose und 0,20 ± 0,15 % Neuraminsäure,
   b) einen Sedimentationskoeffizienten $S_{20,w}^0$ von 2,9 ± 0,2 S,
   c) ein in der Ultrazentrifuge bestimmtes Molekulargewicht von 30 700 ± 3 200,
   d) einen Extinktionskoeffizienten $E_{1cm}^{1\%}$ (280 nm) von 14,2 ± 1,0,
   e) eine elektrophoretische Beweglichkeit im Bereich des Albumins und
   f) einen isoelektrischen Punkt von 4,4 ± 0,1.
2. Verfahren zur Gewinnung oder Isolierung des Proteins nach Anspruch 1, dadurch gekennzeichnet, daß ein Extrakt aus menschlichen Plazenten mindestens einer der folgenden Maßnahmen unterworfen wird :
   a) Fällung des Proteins $PP_{15}$ mit Ammoniumsulfat bei pH 7 und 30-60 % Sättigung ;
   b) Fällung des Proteins $PP_{15}$ mit einer wasserlöslichen Acridinbase bei einem pH-Wert zwischen 4 und 9 und einer Konzentration der Base von 0,2-0,8 g/100 ml ;
   c) Euglobulinfällung bei einem pH-Wert von 5-6 in einer verdünnten Salzlösung, wobei Begleitproteine abgetrennt werden, aber $PP_{15}$ nicht präzipiert wird ;
   d) präparative Zonenelektrophorese und Gewinnung der Fraktion mit der Beweglichkeit von Albumin ;
   e) Gelfiltration, wobei Proteine im Molekulargewichtsbereich von 10 000 bis 50 000 gewonnen werden ;
   f) Adsorption an einem schwach basischen Ionenaustauscher und Elution des Proteins $PP_{15}$ ;
   g) Chromatographie an Hydroxylapatit bei pH 6,0 bis 8,0 in einem Phosphatpuffer, wobei $PP_{15}$ nicht absorbiert wird.
3. Verwendung des Proteins nach Anspruch 1 zur Gewinnung eines Antiserums zum Nachweis und zur Bestimmung dieses Proteins.
4. Protein nach Anspruch 1 zur Verwendung als immunsuppressives Agens.

# 0 033 000

**Ansprüche** (für den Vertragsstaat AT)

1. Verfahren zur Gewinnung oder Isolierung eines al $PP_{15}$ bezeichneten Proteins, das
   a) einen Kohlenhydratanteil von $3,35 \pm 0,9\,\%$, bestehend aus $2,8 \pm 0,5\,\%$ Hexosen, $0,3 \pm 0,2\,\%$ Hexosaminen, $0,05 \pm 0,05\,\%$ Fucose und $0,20 \pm 0,15\,\%$ Neuraminsäure
   b) einen Sedimentationskoeffizienten $_{20,w}^{0}$ von $2,9 \pm 0,2$ S,
   c) ein in der Ultrazentrifuge bestimmtes Molekulargewicht von $30\,700 \pm 3\,200$,
   d) einen Extinktionskoeffizienten $E_{1cm}^{1\%}$ (280 nm) von $14,2 \pm 1,0$,
   e) eine elektrophoretische Beweglichkeit im Bereich des Albumins und
   f) einen isoelektrischen Punkt von $4,4 \pm 0,1$ .
hat, dadurch gekennzeichnet, daß ein Extrakt aus menschlichen Plazenten mindestens einer der folgenden Maßnahmen unterworfen wird :
   a) Fällung des Proteins $PP_{15}$ mit Ammoniumsulfat bei pH 7 und 30-60 % Sättigung ;
   b) Fällung des Proteins $PP_{15}$ mit einer wasserlöslichen Acridinbase bei einem pH-Wert zwischen 4 und 9 und einer Konzentration der Base von 0,2-0,8 g/100 ml ;
   c) Euglobulinfällung bei einem pH-Wert von 5-6 in einer verdünnten Salzlösung, wobei Begleitproteine abgetrennt werden, aber $PP_{15}$ nicht präzipiert wird ;
   d) präparative Zonenelektrophorese und Gewinnung der Fraktion mit der Beweglichkeit von Albumin ;
   e) Gelfiltration, wobei Proteine im Molekulargewichtsbereich von $10\,000$ bis $50\,000$ gewonnen werden ;
   f) Adsorption an einem schwach basischen Ionenaustauscher und Elution des Proteins $PP_{15}$ ;
   g) Chromatographie an Hydroxylapatit bei pH 6,0 bis 8,0 in einem Phosphatpuffer, wobei $PP_{15}$ nicht absorbiert wird.

2. Verwendung des Proteins nach Anspruch 1 zur Gewinnung eines Antiserums zum Nachweis und zur Bestimmung dieses Proteins.

3. Protein nach Anspruch 1 zur Verwendung als immunsuppressives Agens.


**Claims** (for the Contracting States : CH, DE, FR, GB, IT, LI)

1. Protein $PP_{15}$ characterized by
   a) a content of carbohydrates of $3.35 \pm 0.9\,\%$, of which are $2.8 \pm 0.5\,\%$ hexoses, $0.3 \pm 0.2\,\%$ hexosamines, $0.05 \pm 0.05\,\%$ fucose and $0.20 \pm 0.15\,\%$ neuraminic acid,
   b) a sedimentation coefficient $s_{20w}^{0}$ of $2.9 \pm 0.2$ S,
   c) a molecular weight of $30,700 \pm 3,200$ determined in an ultracentrifuge,
   d) an extinction coefficient $E_{1cm}^{1\%}$ (280 nm) of $14.2 \pm 1.0$,
   e) an electrophoretic mobility in the range of albumin and
   f) an isoelectric point of $4.4 \pm 0.1$.

2. Process for preparing or isolating the protein $PP_{15}$ according to claim 1, which comprises subjecting an extract obtained from human placenta to at least one of the following measures :
   a) precipitating the protein $PP_{15}$ with ammonium sulfate at pH 7 and at a saturation of 30 to 60 % ;
   b) precipitating the protein $PP_{15}$ with a water-soluble acridine base at a pH-value of between 4 and 9 and a concentration of the base of from 0.2 to 0.8 g/100 ml ;
   c) euglobulin precipitation at a pH from 5 to 6 in a diluted salt solution, whereby accompanying proteins are separated and $PP_{15}$ is not precipitated ;
   d) preparative zone electrophoresis and isolating the fraction with the mobility of albumin ;
   e) gel-filtration, whereby proteins in the molecular weight range of from 10,000 to 50,000 are isolated ;
   f) adsorbing $PP_{15}$ on a weakly basic ion exchanger and eluting it ;
   g) chromatography on hydroxyapatite at a pH of 6.0 to 8.0 in a phosphate buffer, whereby $PP_{15}$ is not adsorbed.

3. Use of the protein according to claim 1 for preparing an antiserum for detecting and determining this protein.

4. The protein according to claim 1 for use as an immunsuppressive agent.


**Claims** (for the Contracting State AT)

1. Process for preparing or isolating a protein designated $PP_{15}$, which has
   a) a content of carbohydrates of $3.35 \pm 0.9\,\%$, of which are $2.8 \pm 0.5\,\%$ hexoses, $0.3 \pm 0.2\,\%$ hexosamines, $0.05 \pm 0.05\,\%$ fucose and $0.20 \pm 0.15\,\%$ neuraminic acid,
   b) a sedimentation coefficient $s_{20w}^{0}$ of $2.9 \pm 0.2$ S,
   c) a molecular weight of $30,700 \pm 3,200$ determined in an ultra-centrifuge,
   d) an extinction coefficient $E_{1cm}^{1\%}$ (280 nm) of $14.2 \pm 1.0$,

e) an electrophoretic mobility in the range of albumin and

f) an isoelectric point of 4.4 ± 0.1,

which process comprises subjecting an extract obtained from human placenta to at least one of the following measures ;

a) precipitating the protein $PP_{15}$ with ammonium sulfate at pH 7 and at a saturation of 30 to 60 % ;

b) precipitating the protein $PP_{15}$ with a water-soluble acridine base at a pH-value of between 4 and 9 and a concentration of the base of from 0.2 to 0.8 g/100 ml ;

c) euglobulin precipitation at a pH from 5 to 6 in a diluted salt solution, whereby accompanying proteins are separated and $PP_{15}$ is not precipitated ;

d) preparative zone electrophoresis and isolating the fraction with the mobility of albumin ;

e) gel-filtration, whereby proteins in the molecular weight range of from 10,000 to 50,000 are isolated ;

f) adsorbing $PP_{15}$ on a weakly basic ion exchanger and eluting it ;

g) chromatography on hydroxyapatite at a pH of 6.0 to 8.0 in a phosphate buffer, whereby $PP_{15}$ is not adsorbed.

2. Use of the protein according to claim 1 for preparing an antiserum for detecting and determining this protein.

3. The protein according to claim 1 for use as an immunsuppressive agent.


**Revendications** (pour les Etats contractants : CH, DE, FR, GB, IT, LI)

1. Protéine $PP_{15}$ caractérisée par :

a) une teneur en hydrates de carbone de 3,35 ± 0,9 %, constituée de 2,8 ± 0,5 % d'hexoses, 0,3 ± 0,2 % d'hexosamines, 0,05 ± 0,05 % de fucose et 0,20 ± 0,15 % d'acide neuraminique,

b) un coefficient de sédimentation $S_{20,w}^0$ de 2,9 ± 0,2 S,

c) une masse moléculaire déterminée par ultracentrifugation de 30 700 ± 3 200,

d) un coefficient d'extinction $E_{1cm}^{1\%}$ (280 nm) de 14,2 ± 1,0,

e) une mobilité électrophorétique dans la région de l'albumine et

f) un point isoélectrique de 4,4 ± 0,1.

2. Procédé pour l'obtention ou l'isolement de la protéine selon la revendication 1, caractérisé en ce qu'on soumet un extrait de placentas humains à l'une des mesures suivantes :

a) précipitation de la protéine $PP_{15}$ avec du sulfate d'ammonium à un pH de 7 et une saturation de 30 à 60 % ;

b) précipitation de la protéine $PP_{15}$ avec une base d'acridine soluble dans l'eau à une valeur de pH comprise entre 4 et 9 et une concentration de la base de 0,2 à 0,8 g/100 ml ;

c) précipitation des euglobulines à une valeur de pH de 5 à 6 dans une solution saline diluée, les protéines secondaires étant séparées, mais la protéine $PP_{15}$ n'étant pas précipitée ;

d) électrophorèse préparative de zones et obtention de la fraction ayant la mobilité de l'albumine ;

e) filtration sur gel pour recueillir les protéines ayant une masse moléculaire comprise entre 10.000 et 50.000 ;

f) adsorption sur un échangeur d'ions faiblement basique et élution de la protéine $PP_{15}$ ;

g) chromatographie sur hydroxylapatite à un pH de 6,0 à 8,0 dans un tampon au phosphate, $PP_{15}$ n'étant pas absorbée.

3. Utilisation de la protéine selon la revendication 1 pour la production d'un antisérum pour la détection et le dosage de cette protéine.

4. Protéine selon la revendication 1, pour utilisation comme agent immunosuppresseur.


**Revendications** (pour l'Etat contractant AT)

1. Procédé pour l'obtention ou l'isolement d'une protéine appelée $PP_{15}$ qui a :

a) une teneur en hydrates de carbone de 3,35 ± 0,9 %, constituée en 2,8 ± 0,5 % d'hexoses, 0,3 ± 0,2 % d'hexosamines, 0,05 ± 0,05 % de fucose et 0,20 ± 0,15 % d'acide neuraminique,

b) un coefficient de sédimentation $S_{20,w}^0$ de 2,9 ± 0,2 S,

c) une masse moléculaire déterminée par ultracentrifugation de 30 700 ± 3 200,

d) un coefficient d'extinction $E_{1cm}^{1\%}$ (280 nm) de 14,2 ± 1,0,

e) une mobilité électrophorétique dans la région de l'albumine et

f) un point isoélectrique de 4,4 ± 0,1,

caractérisé en ce qu'on soumet un extrait de placentas humains à au moins l'une des mesures suivantes :

a) précipitation de la protéine $PP_{15}$ avec du sulfate d'ammonium à un pH de 7 et une saturation de 30 à 60 % ;

b) précipitation de la protéine $PP_{15}$ avec une base d'acridine soluble dans l'eau à une valeur de pH comprise entre 4 et 9 et une concentration de la base de 0,2 à 0,8 g/100 ml ;

c) précipitation des euglobulines à une valeur de pH de 5 à 6 dans une solution saline diluée, les

**0 033 000**

protéines secondaires étant séparées, mais PP$_{15}$ n'étant pas précipitée ;

d) électrophorèse préparative de zones et obtention de la fraction ayant la mobilité de l'albumine ;

e) filtration sur gel pour obtenir les protéines ayant une masse moléculaire de 10 000 à 50 000 ;

f) adsorption sur un échangeur d'ion faiblement basique et élution de la protéine PP$_{15}$ ;

g) chromatographie sur hydroxylapatite à un pH de 6,0 à 8,0 dans un tampon au phosphate, PP$_{15}$ n'étant pas absorbée.

2. Utilisation de la protéine selon la revendication 1 pour la production d'un antisérum pour la détection et le dosage de cette protéine.

3. Protéine selon la revendication 1 pour utilisation comme agent immunosuppresseur.